# EUROPEAN PATENT APPLICATION

(11) **EP 1 600 109 A1**
(43) Date of publication of application: **30.11.2005**
(21) Application number: 04708862.0
(22) Date of filing: 06.02.2004
(51) Int. Cl.: A61B 17/12

(54) **DEVICE FOR BLOCING BLOOD VESSEL**

(30) Priority: 07.02.2003 JP 2003031490
(71) Applicant: Medgel Corporation, Kyoto-shi, Kyoto 612-8043 (JP)
(72) Inventor: TABATA, Yasuhiko, Uji-shi, Kyoto 6110024 (JP); MIYAMOTO, Susumu, Kyoto 606-0926 (JP); YAMADA, Keisuke 125-206, Aioi-cho, Kyoto 6020827 (JP)
(74) Representative: Vossius & Partner
(86) International application number: PCT/JP2004/001283
(87) International publication number: WO 2004/069058

(57) **Abstract**

Disclosed is a device for occlusion of blood vessel comprising a metallic coil, a sustained-release element and a cell growth factor. Examples of the cell growth factor include basic fibroblast growth factor (bFGF), acidic fibroblast growth factor (aFGF), platelet-derived growth factor (PDGF), transforming growth factor β1 (TGC-β1), vascular endothelia growth factor (VEGF) and connective tissue growth factor (CTGF). Examples of the sustained-release element include a hydrophilic hydrogel. By using the device for occlusion of blood vessel according to the present invention, a blood vessel site such as aneurysm is occluded, and preferably, organization may be induced *in vivo.*

## Description

### Technical Field

The present invention relates to a device for occlusion of blood vessel to be used for blood vessel embolotherapy which is effective for treatment of aneurysm.

### Background Art

Recently, as a minimally invasive treatment method for aneurysm, the so-called intravascular operation has been proposed where aneurysm is occluded by stationing a substance for blood vessel occlusion in the aneurysm using a catheter. Various substances have been proposed for occlusion of blood vessel, including, cellulose sponge (Japanese Patent Laid-Open No. 02/031,762), gelatin particles (Japanese Patent Laid-Open No. 60/222,045), spiral coil (Japanese Patent Laid-Open No. 09/510,637), twist-shaped coil mainly made of metal (Japanese Patent No. 3,016,418), rod made of hydrophilic resin (Japanese Patent Laid-Open No. 11/047,138), coil to which fibrous material is attached (Japanese Patent Laid-Open Nos. 06/506,622 and 06/510,938).

Among them, a substance commonly used for occlusion of the blood vessel in the treatment of aneurysm is "micro-coil mainly made of platinum-type alloy" and such coils in various shapes and characteristics have been proposed. The micro-coil is attached to the distal end of a catheter and made to reach a predetermined site in the blood vessel (e.g., Japanese Patent Laid-Open Nos. 08/505,105 and 07/503,674), where the micro-coil is separated from the catheter in the aneurysm either mechanically or electrically or by means of hydraulic pressure, and is placed at that site. As a result, the placed micro-coil promotes generation of thrombus whereby aneurysm is occluded by the thrombus and cured.

Besides treatment of aneurysm, a blood vessel occlusion method using a coil has also been used as a method for treatment of ductus arteriosus, arteriovenous fistula, cerebral arteriovenous malformation and other blood vessel symptoms. In addition, one of the methods for the treatment of tumor involves plugging the feeding artery of tumor to cause recession or regression of the tumor.

In conducting such a blood vessel occluding treatment, a medical wire is prepared in which a metallic coil is attached at the front end of a guide wire, then the front end is inserted into the target site of the blood vessel, where the metallic coil made of a substance for inducing occlusion of blood vessel is separated from the guide wire and placed in the target site. However, embolus by thrombus may cause some problems, such as shrinking of thrombus by blood pressure, disappearance of occlusion effect by transfer of thrombus in blood vessel and formation of aneurysm or rupture at another site. Therefore, there has been a demand in the art to develop a device for blood vessel occlusion which is capable of occluding the blood vessel more effectively.

An object of the present invention is to provide a device for blood vessel occlusion which can occlude aneurysm of the blood vessel, and preferably can induce organization *in vivo.*

### Disclosure of the Invention

The present invention provides a device for occlusion of blood vessel comprising a metallic coil, a sustained-release element and a cell growth factor. The present invention also provides a method for plugging the blood vessel comprising providing a device for occlusion of blood vessel at a site of a patient's blood vessel to be plugged, wherein the devise comprises a metallic coil, a sustained-release element and a cell growth factor.

Preferably, the cell growth factor is selected from the group consisting of basic fibroblast growth factor (bFGF), acidic fibroblast growth factor (aFGF), platelet-derived growth factor (PDGF), transforming growth factor β1 (TGC-β1), vascular endothelia growth factor (VEGF) and connective tissue growth factor (CTGF). Also preferably, the sustained-release element is hydrophilic hydrogel.

### Brief Description of the Drawings

Fig. 1 is a photographic external view of the tissue connecting carotid artery with aneurysm in an aneurysm model animal subjected to a treatment with a metallic micro-coil of the present invention.
Fig. 2 is a photographic view, observed from the artery lumen, of the tissue connecting carotid artery with aneurysm in an aneurysm model animal subjected to a treatment with a metallic micro-coil of the present invention.

### Detailed Description of the Invention

The device for occlusion of blood vessel according to the present invention is characterized in comprising a metallic coil, a sustained-release element and a cell growth factor. The device for occlusion of blood vessel according to the present invention is applied to a predetermined site of blood vessel or, particularly, to aneurysm, where it is able to plug the blood vessel at that site. Preferably, the device for occlusion of blood vessel according to the present invention is able to induce organization in vivo. As used herein, the term organization does not mean that thrombus is generated around the material introduced into the blood vessel, but rather means that the thrombus is substituted by fibrous tissue. When this type of tissue is formed in the aneurysm and is able to completely fill the inside of the aneurysm, the aneurysm site will become non-breakable and aneurysm will not be re-generated, whereby a complete curing of aneurysm can be achieved.

When the device of the present invention is placed in the internal carotid aneurysm of an aneurysm model rabbit, the organization of tissue is induced and the blood vessel will be coated with vascular endothelial cells, resulting in the occlusion of the aneurysm within three weeks.

Examples of the materials used for the metallic coil in the device of the present invention include platinum, gold, tungsten, stainless steel and an alloy thereof. The metallic coil may be prepared by forming a metal wire into a spiral form. The coil may be in a single helix or in a double or triple helix. Further, metal or fiber branches or loops may be attached to the coil, and when a secondary coil is extruded from a guide wire, the metallic coil may be assembled into a random three-dimensional structure. Metallic coils for plugging with Various characteristics and shapes are commercially available, and any of them may be advantageously used in the present invention. For example, an example of a coil suitable for plugging cerebral aneurysm is a micro-coil in which a wire of 0.05 to 0.15 mm diameter is formed into a primary coil of 0.3 to 0.9 mm diameter, which in turn is formed into a helical secondary coil.

Examples of the sustained-release element are hydrophilic hydrogels comprising a water-soluble polysaccharide such as alginic acid, hyaluronic acid and carboxymethyl cellulose, a protein such as gelatin and pectin, a water-soluble synthetic polymer formed from polyvinyl alcohol, polyacrylic acid and polymalic acid, or collagen, or a mixture or a complex thereof. For example, a bi-functional reagent such as glutaraldehyde is added to an aqueous solution of gelatin to allow for cross-linking, and then the mixture is swollen by water or a buffer to form a hydrogel. A metallic coil is dipped in a mixed aqueous solution of gelatin and glutaraldehyde to form a metallic coil carrying a sustained-release element adhered thereto. Then the coil is allowed to stand, whereby gelatin is cross-linked and the resulted sustained-release element is fixed to the metal coil to prepare a device comprising the metallic coil and the sustained-release element. If necessary, unreacted glutaraldehyde may be washed out. Cross-linking of gelatin may also be conducted by irradiation of ultraviolet ray, gamma ray or electronic ray or by a thermal dehydration. The metallic coil is dipped into an aqueous solution of gelatin, and then dried by vacuum or freeze drying. Then the above-mentioned operation is applied to provide a cross-linked gelatin. In any of the above-mentioned cross-linking methods, the degree of cross-linking of the gelatin hydrogel may be controlled by changing the cross-linking conditions; thus the degradation properties of the hydrogel in vivo may be controlled. The sustained-release pattern of the cell growth factor impregnated in the hydrogel may be controlled by changing the degradation properties of the hydrogel.

Next, an aqueous solution of a cell growth factor is impregnated into a device comprising the metallic coil and the sustained-release element prepared as above. In the present invention, the cell growth factor means a substance having an activity of promoting the organization of tissues, including a protein, a peptide, a nucleic acid and a low-molecular drug. Examples of the preferred cell growth factor include basic fibroblast growth factor (bFGF), acidic fibroblast growth factor (aFGF), platelet-derived growth factor (PDGF), transforming growth factor β1 (TGC-β1), vascular endothelia growth factor (VEGF) and connective tissue growth factor (CTGF). Those growth factors are commercially available or may be recombinantly produced using a gene coding for the growth factor protein. In addition, in the present invention it is also possible to use a low-molecular substance which promotes the secretion of such a cell growth factor (such as statine and rapamycin) or a gene coding for a cell growth factor. One of those substances may be used alone or two or more thereof may be used in combination.

When the device comprising a metallic coil, a sustained-release element and a cell growth factor prepared as above is applied to the site of the blood vessel to be plugged, the cell growth factor contained therein will be gradually released from the sustained-release element over a long time, which can promote the growth of fibroblast or the like and induce organization by fibrous tissue. As a result of release of a cell growth factor in a predetermined concentration for a long time, an appropriate organization is induced and occlusion of blood vessel is promoted. Especially in the treatment of aneurysm, it is believed that the ideal treatment of aneurysm can be achieved by filling the aneurysm with autologous tissue. When fibroblast tissue is formed in the aneurysm, such a site becomes non-breakable and the aneurysm is stably plugged for a long period of time, namely, the organization takes place. At-this stage, the lumen surface of the blood vessel of the aneurysm filled with tissue at the site of originally connecting to the blood vessel is covered by vascular endothelial cells which are of the same type as those on the surrounding natural blood vessel.

All patents and references which are explicitly cited in the present specification are hereby incorporated by reference. All of the contents mentioned in the specification and the drawings of Japanese Patent Application No. 2003/031,490, which is a basic application for claiming a priority of the present application, are hereby incorporated by reference.

The present invention will be illustrated in more detail by way of the following Examples, although the present invention is not limited by such Examples.

### Examples

### Example 1: Preparation of device for embolus of blood vessel

A micro-coil made of platinum (GDC 18 soft, product number 351304-4; and GDC 10 soft, product number 340304-4; both manufactured by Boston Scientific Japan) was used as a metallic coil.

A 25% aqueous solution of glutaraldehyde (125 µl or 80 µl) was quickly added (within one minute) to 40 ml of a 5% aqueous solution of gelatin with stirring to give an aqueous solution of gelatin-glutaraldehyde. Immediately after addition of the aqueous solution of glutaraldehyde, the metallic micro-coil was dipped in this solution, and allowed to stand at 4°C for 12 hours so that gelatin was cross-linked. The metallic micro-coil to which the cross-linked gelatin hydrogel was adhered was cut out from the gelatin hydrogel and allowed to added to an aqueous solution of glycine at 37°C for 1 hour to block the residual aldehyde groups in the hydrogel. Then the micro-coil was washed with water twice, freeze-dried, and sterilized with ethylene oxide gas.

Then, 11 µl of an aqueous solution of bFGF (containing 100 µg) was added dropwise on the metallic micro-coil carrying cross-linked gelatin hydrogel prepared as above, and allowed to stand at 4°C for 8 hours so that the freeze-dried cross-linked gelatin hydrogel was swollen with the aqueous solution of bFGF. In this way, the metallic micro-coil to which bGFG-impregnated gelatin hydrogel was adhered was prepared as a device for blood vessel embolus.

### Example 2: Embolus study in an aneurysm model animal

According to a method of Guglielmi, et al. (Guglielmi, G., et al., *J*. *Neurosurgery,* 75, 1 (1991)), the inner jugular vein of a rabbit was excised over a length of 8 mm, and transplanted onto the internal carotid artery to prepare a pocket-shaped model of aneurysm having a depth of about 5 mm. The device for blood vessel embolus prepared in Example 1 or, as a control, a metallic micro-coil carrying the hydrogel but not impregnated with bFGF, was placed in the resulting model aneurysm and the side of the aneurysm opposite from the blood vessel was closed by a suture.

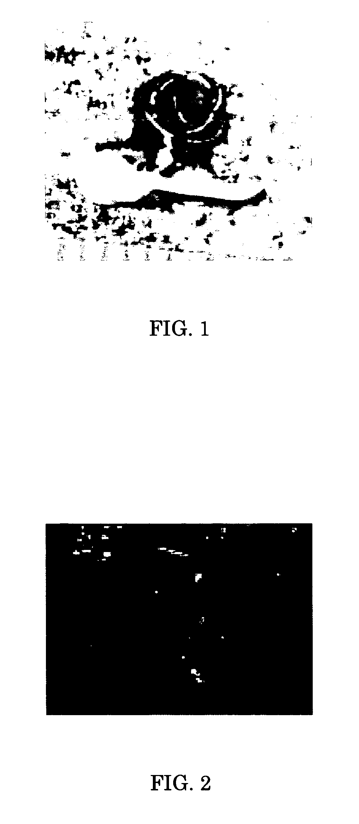

After 3 weeks, the rats were sacrificed to examine the condition of the aneurysm and organization by fibrous tissue in the aneurysm. In the group where the metallic micro-coil carrying bFGF-impregnated gelatin hydrogel was used, the aneurysm was completely filled with blood vessel tissue containing the coil, and the lumen surface of the artery portion connecting the internal carotid artery and the aneurysm was covered by vascular endothelial cells which were of the same type as those of natural blood vessel (Figs. 1 and 2). Observation of tissue slices inside the aneurysm further confirmed that inside of the aneurysm was filled with the tissue. On the contrary, in the group where the metallic coil carrying the hydrogel without bFGF, the inside of the aneurysm was filled only with thrombus and neither blood vessel tissue nor endothelial cells were observed.

## Claims

1. A device for occlusion of blood vessel comprising a metallic coil, a sustained-release element and a cell growth factor.

2. The device according to claim 1, wherein the cell growth factor is selected from the group consisting of basic fibroblast growth factor (bFGF), acidic fibroblast growth factor (aFGF), platelet-derived growth factor (PDGF), transforming growth factor β1 (TGC-β1), vascular endothelia growth factor (VEGF) and connective tissue growth factor (CTGF).

3. The device according to claim 1 or 2, wherein the sustained-release element is a hydrophilic hydrogel.

4. A method for plugging the blood vessel comprising providing a device for occlusion of blood vessel at a site of a patient's blood vessel to be plugged, said device comprising a metallic coil, a sustained-release element and a cell growth factor.

5. The method according to claim 4, wherein the cell growth factor is selected from the group consisting of basic fibroblast growth factor (bFGF), acidic fibroblast growth factor (aFGF), platelet-derived growth factor (PDGF), transforming growth factor β1 (TGC-β1), vascular endothelia growth factor (VEGF) and connective tissue growth factor (CTGF).

6. The method according to claim 4 or 5, wherein the sustained-release element is a hydrophilic hydrogel.
